# EUROPÄISCHE PATENTSCHRIFT

(11) **EP 2 867 208 B1**
(45) Veröffentlichungstag und Bekanntmachung des Hinweises auf die Patenterteilung: **24.08.2016**
(21) Anmeldenummer: 13730578.5
(22) Anmeldetag: 24.06.2013
(51) Int. Cl.: C07D 213/61

(54) **VERFAHREN ZUR HERSTELLUNG VON 2,2-DIFLUORETHYLAMIN-DERIVATEN DURCH ALKYLIERUNG VON 2,2-DIFLUORETHYLAMIN**
PROCESS FOR PREPARING 2,2-DIFLUOROETHYLAMINE DERIVATIVES BY ALKYLATING 2,2-DIFLUOROETHYLAMINE
PROCÉDÉ DE PRODUCTION DE DÉRIVÉS DE 2,2-DIFLUOROÉTHYLAMINE PAR ALKYLATION DE 2,2-DIFLUOROÉTHYLAMINE

(30) Priorität: 29.06.2012 EP 12174277
(43) Veröffentlichungstag der Anmeldung: 06.05.2015
(73) Patentinhaber: Bayer CropScience AG, 40789 Monheim am Rhein (DE)
(72) Erfinder: FUNKE, Christian, 42799 Leichlingen (DE); LUI, Norbert, 51519 Odenthal (DE); WARSITZ, Rafael, 45136 Essen (DE); SCHNATTERER, Albert, 51373 Leverkusen (DE)
(74) Vertreter: BIP Patents
(86) Internationale Anmeldenummer: PCT/EP2013/063116
(87) Internationale Veröffentlichungsnummer: WO 2014/001245

(56) Entgegenhaltungen:
- WO-A1-01/46170
- WO-A1-2007/115644

## Beschreibung

Die vorliegende Erfindung betrifft ein Verfahren (Prozess) zur Herstellung bestimmter 2,2-Difluorethylamin-Derivate ausgehend von 2,2-Difluorethylamin.

2,2-Difluorethylamin-Derivate sind nützliche Zwischenstufen bei der Herstellung agrochemischer Wirkstoffe (siehe WO 2007/115644). Es sind verschiedene Verfahren zur Herstellung von 2,2-Difluorethylamin-Derivaten bekannt.

WO 2009/036900 beschreibt beispielsweise ein Verfahren zur Herstellung von 2,2-Difluorethylamin-Derivaten durch Amid-Hydrierung von N-[(6-Chlorpyridin-3-yl)methyl]-2,2-difluoracetamid (Schema 1).

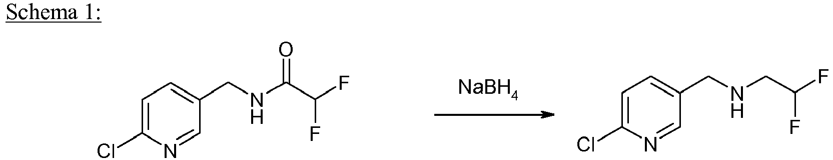

Nachteilig bei diesem Verfahren ist der Einsatz komplexer Hydride wie Natriumborhydrid, weil Hydride teuer und sicherheitstechnisch aufwendig zu verwenden sind.

WO 2009/036901 beschreibt die Reduktion von N-(6-Chlorpyridin-3-yl)methylen-2,2-difluorethanamin durch Wasserstoff (Schema 2).

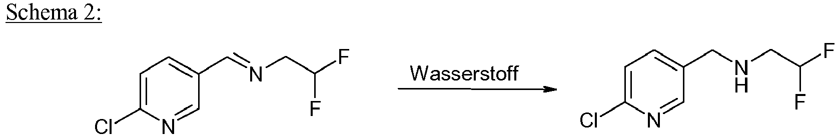

Nachteilig bei diesem Verfahren ist der Einsatz von Wasserstoff, weil auch hier die Verwendung von Wasserstoff sicherheitstechnisch sehr aufwendig ist.

WO 2011/157650 beschreibt die Herstellung der 2,2-Difluorethanamin-Derivate ausgehend von 2,2-Difluor-1-halogenethanen mit primären Aminen in Anwesenheit von organischen Basen (Schema 3).

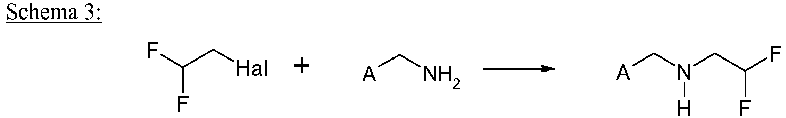

Nachteilig an diesem Verfahren ist, dass die Reaktion in einem Hochdruckapparat (Autoklav) durchgeführt werden muss.

Die Patentpublikation WO 2007/115644, die sich mit der Herstellung von insektizid wirksamen 4-Aminobut-2-enolidverbindungen befasst, beschreibt die Herstellung von Verbindungen der allgemeinen Formel A-CH₂-NH-R¹, in der A für spezielle Heterocyclen und R¹ für Halogenalkyl steht, durch Alkylierung des Stickstoffs (Schema 4).

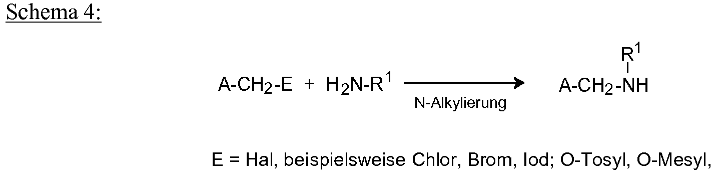

Konkret beschreibt WO2007/115644 die Herstellung von N-[(6-Chlorpyridin-3-yl)methyl]-2,2-difluorethan-1-amin (Verbindung (3), das ausgehend von 2-Chlor-5-chlormethyl-pyridin (Verbindung (2)) und 2,2-Difluorethan-1-amin (Verbindung 1)) in Gegenwart von Triethylamin synthetisiert wird (siehe Schema 5)). Die Verbindungen (1), (2) und Triethylamin werden dabei in äquimolaren Mengen eingesetzt. Das gewünschte Produkt wird in einer Ausbeute von 53 % erhalten.

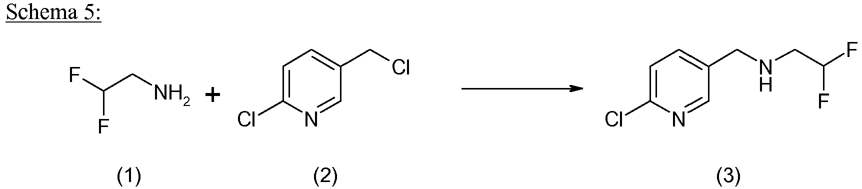

Das in WO 2007/116544 beschriebene Verfahren zur Herstellung von Verbindungen der Formel A-CH₂-NH-R¹, in der A für spezielle Heterocyclen und R¹ für Halogenalkyl steht, ist nachteilig, da es während der Umsetzung zu Mehrfachalkylierungen des Stickstoffs kommen kann. Dies führt zu einem Ausbeuteverlust, was auch an der Ausbeute des konkret genannten Beispiels zu erkennen ist. Die Ausbeute betrug lediglich 53 %. Diese Mehrfachalkylierungen können nur durch den Einsatz eines großen Überschusses an Amin reduziert werden. Allerdings ist die destillative Wiedergewinnung des kostspieligen Amins im Regelfall aufwendig und nicht verlustfrei.

Wegen der Bedeutung von 2,2-Difluorethylamin-Derivaten als Bausteine zur Synthese agrochemischer Wirkstoffe ist es jedoch notwendig, ein Verfahren zu finden, das großtechnisch und kostengünstig eingesetzt werden kann. Auch ist es erstrebenswert die speziellen 2,2-Difluorethylamin-Derivate mit hoher Ausbeute und hoher Reinheit zu erhalten, so dass die Zielverbindung vorzugsweise keiner weiteren möglicherweise komplexen Aufreinigung unterzogen werden muss.

Es wurde nun ein Verfahren zur Herstellung bestimmter 2,2-Difluorethylamin-Derivate gefunden, das die Nachteile der bekannten Verfahren vermeidet und überdies hinaus noch einfach und kostengünstig durchzuführen ist, sodass es großtechnisch eingesetzt werden kann.

Die vorliegende Erfindung betrifft somit ein Verfahren zur Herstellung bestimmter 2,2-Difluorethylamin-Derivate der allgemeinen Formel (III) 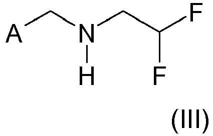 in der
- A: für einen Rest Pyrid-2-yl, Pyrid-4-yl oder Pyrid-3-yl steht, der jeweils in 6-Position durch Fluor, Chlor, Brom, Methyl, Trifluormethyl oder Trifluormethoxy substituiert sein kann, oder für Pyridazin-3-yl steht, das in 6-Position durch Chlor oder Methyl substituiert sein kann, oder für einen Rest Pyrazin-3-yl, 2-Chlor-pyrazin-5-yl oder 1,3-Thiazol-5-yl steht, der jeweils in 2-Position durch Chlor oder Methyl substituiert sein kann, oder für einen Rest Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, der durch Fluor, Chlor, Brom, Cyano, Nitro, gegebenenfalls durch Fluor und/oder Chlor substituiertes C₁-C₄-Alkyl, gegebenenfalls durch Fluor und/oder Chlor substituiertes C₁-C₃-Alkylthio, oder gegebenenfalls durch Fluor und/oder Chlor substituiertes C₁-C₃-Alkylsulfonyl, substituiert sein kann, oder für ein Pyrid-3-yl der folgenden Formel 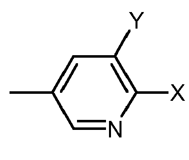 steht, in der
- X: für Halogen, C₁-C₁₂-Alkyl (bevorzugt , C₁-C₆-Alkyl) oder C₁-C₁₂-Halogenalkyl (bevorzugt C₁-C₆-Halogenalkyl) steht und
- Y: für Halogen, C₁-C₁₂-Alkyl (bevorzugt , C₁-C₆-Alkyl), C₁-C₁₂-Halogenalkyl (bevorzugt C₁-C₆-Halogenalkyl), C₁-C₁₂-Halogenalkoxy (bevorzugt C₁-C₆-Halogenalkoxy), Azido oder Cyan steht,
in dem 2,2-Difluorethylamin der Formel (I) 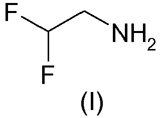 mit einem Halogenid der allgemeinen Formel (II) 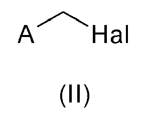 in der Hal für Cl, Br oder Jod steht,
in Gegenwart von Diisopropylethylamin umgesetzt wird.

Die erfindungsgemäße Reaktion ist in Schema 6 dargestellt.

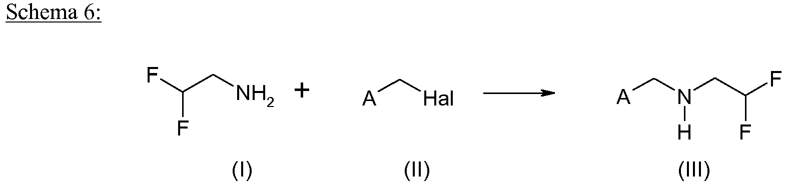

Die gewünschten 2,2-Difluorethylamin-Derivate der allgemeinen Formel (III) werden mit dem erfindungsgemäßen Verfahren mit guten Ausbeuten und in hoher Reinheit erhalten. Die gewünschten Verbindungen werden dabei in einer Reinheit erhalten, welche eine umfangreiche Aufarbeitung des Reaktionsprodukts im Allgemeinen nicht erforderlich macht.

Mit dem erfindungsgemäßen Verfahren können signifikant bessere Ausbeuten erzielt werden als mit dem in WO2007/115644 beschriebenen Verfahren, in dem Triethylamin als tertiäre Stickstoff-Base eingesetzt wird.

Im Rahmen der vorliegenden Erfindung wird unter einem Derivat ein von dem bezeichneten organischen Grundgerüst (Baustein) abgeleiteter Stoff ähnlicher Struktur bezeichnet, d.h. unter einem 2,2-Difluorethylamin-Derivat wird insbesondere eine Verbindung verstanden, welche einen 2,2-Difluorethylamin-Baustein umfasst.

Bevorzugt wird eine Verbindung der allgemeinen Formel (II) in der Hal für Chlor und Brom steht, eingesetzt. Besonders bevorzugt ist die Verbindung der Formel (II) in der Hal für Chor steht. Weiterhin bevorzugt werden Verbindungen der Formel (II) im erfindungsgemäßen Verfahren eingesetzt, in der der Rest A ausgewählt ist aus einer Gruppe bestehend aus 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl und 5-Difluormethyl-6-iod-pyrid-3-yl.

Bevorzugte Reste A sind 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl und 5-Difluormethyl-6-chlor-pyrid-3-yl.

Besonders bevorzugte Reste A sind 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl.

Als Verbindung der Formel (II) wird bevorzugt 2-Chlor-(5-chlormethyl)pyridin eingesetzt, sodass N-[(6-Chlorpyridin-3-yl)methyl]-2,2-difluorethan-1-amin erhalten wird.

Das erfindungsgemäße Verfahren erfolgt in Gegenwart der tertiären Stickstoff-Base Diisopropylethylamin.

Das Verfahren erfolgt in Gegenwart einer (d.h. einer oder mehrerer) tertiären Stickstoff-Base. Tertiäre Amine wie Diisopropylethylamin, Tricyclohexylamin oder C₁-C₁₂-Alkylimidazole (z.B. Methylimidazol und Butylimidazol) sind dabei geeignet, wobei Diisopropylethylamin erfindungsgemäß eingesetzt wird. Das Verwenden der Base in der erfindungsgemäßen Reaktion hat den Vorteil, dass die Reaktionslösung nach erfolgter Umsetzung homogen bleibt und dass so das unverbrauchte 2,2-Difluorethylamin (I) einfach und praktisch vollständig abdestilliert und dem Verfahren erneut zugeführt werden kann. Somit kann das Verfahren besonders ressourcenschonend und kostengünstig durchgeführt werden.

Bei Einsatz von Diisopropylethylamin als tertiären Stickstoff-Base lassen sich sehr viel höhere Ausbeuten erzielen als mit anderen tertiären Aminen. Dies ergibt sich auch aus den Beispielen.

Bei Einsatz von C₁-C₁₂-Alkylimidazole als tertiärer Stickstoff-Base lassen sich ebenfalls höhere Ausbeuten erzielen als bei Einsatz von Triethylamin (WO-A-2007/115644). Daher ist auch diese alternative Verfahrensvariante Gegenstand der Erfindung.

Das molare Verhältnis der tertiären Stickstoff-Base (Diisopropylethylamin) zu dem eingesetztem Halogenid der Formel (II) kann beispielsweise im Bereich von etwa 10 bis etwa 0,5 liegen. Vorzugsweise liegt es im Bereich von etwa 8 bis etwa 1, besonders bevorzugt im Bereich von etwa 6 bis etwa 1,1. Der Einsatz größerer Mengen an tertiärer Stickstoff-Base ist grundsätzlich möglich, jedoch unwirtschaftlich.

Im erfindungsgemäßen Verfahren wird 2,2-Difluoramin im Überschuss eingesetzt. Das molare Verhältnis von Halogenid der allgemeinen Formel (II) zum eingesetzten 2,2-Difluorethylamin liegt im Allgemeinen im Bereich von etwa 1 : 1,5 bis etwa 1 : 20. Bevorzugt im Bereich von etwa 1 : 2 bis etwa 1 : 10, besonders bevorzugt von etwa 1 : 2,5 bis etwa 1 : 5.

Da die Edukte flüssige sind, kann das erfindungsgemäße Verfahren ohne ein zusätzliches Lösungsmittel für die Reaktion durchgeführt werden. Selbstverständlich kann auch in Anwesenheit eines Lösungsmittels gearbeitet werden.

Die erfindungsgemäße Reaktion kann in einem weiten Temperaturbereich (z.B. im Bereich von 20°C bis 100°C) durchgeführt werden. Vorzugsweise wird die Umsetzung in einem Temperaturbereich von 35° bis 60°C durchgeführt.

Die Umsetzung wird grundsätzlich unter Normaldruck durchgeführt.

Die Reaktionsdauer der Reaktion ist kurz und liegt im Bereich von etwa 0,5 bis etwa 5 Stunden. Eine längere Reaktionsdauer ist möglich, jedoch wirtschaftlich nicht sinnvoll.

Zur Aufarbeitung des Reaktionsgemisches werden die Überschüsse an eingesetztem 2,2-Difluorethylamin und der tertiären Stickstoff-Base (Diisopropylethylamin) destillativ abgetrennt und stehen für eine weitere Umsetzung zur Verfügung, d.h. sie können für die nächste Partie eingesetzt werden. Nach der Destillation wird normalerweise die Reaktionsmischung mit einem inerten Lösungsmittel wie Toluol oder Butyronitril gelöst und mit Wasser versetzt. Nach pH-Wert-Einstellung der Lösung auf 5,5-6 werden die Phasen getrennt. Das 2,2-Difluorethylamin-Derivat der Formel (III) kann anschließend unter Normaldruck oder im Vakuum, vorzugsweise durch Destillation, isoliert werden. Alternativ kann die organische Phase, in der sich das gewünschte Amin der Formel (III) befindet, direkt für eine weitere Umsetzung eingesetzt werden.

Das tertiäre Amin, d.h. die tertiäre Stickstoff-Base (Diisopropylethylamin), kann über sein Hydrochlorid durch Umsetzung mit einer anorganischen Base aus seinen Salzen freigesetzt und wieder verwendet werden. Eine anorganische Base ist beispielsweise NaOH.

### Beispiele:

Die vorliegende Erfindung wird anhand der nachfolgenden Beispiele näher erläutert, ohne die Erfindung dabei auf diese einzuschränken.

### Beispiel 1 (erfindungsgemäß)

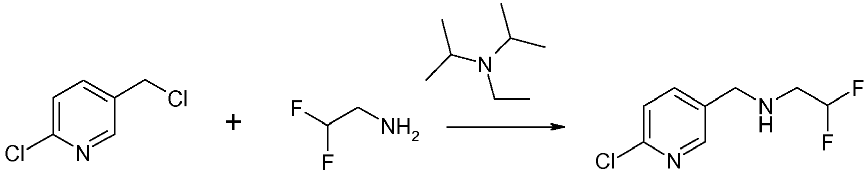

74,4 g (0,90 mol) 2,2-Difluorethylamin (Gehalt: 98%) und 62,6 g (0,48 mol) N, N-Diisopropylethylamin (Gehalt: 99%) werden auf 55°C erhitzt. Zu dieser Mischung werden 49,5 g (0,30 mol) 2-Chlor-(5-chlormethyl)pyridin (Gehalt: 98%) innerhalb von 2,5 Stunden bei dieser Temperatur zugetropft. Die gelbe Lösung läßt man 2 Stunden bei dieser Temperatur nachrühren und destilliert anschließend 72,6 g einer Mischung aus 2,2-Difluorethylamin und N, N-Diisopropylethylamin ab.

Nach GC-Methode mit externem Standard entspricht die Wiedergewinnung an 2,2-Difluorethylamin-Überschuss 0,559 mol (95%) und die Wiedergewinnung N, N-Diisopropylethylamin-Überschuss 0,17 mol (93%).

Der Rückstand wird mit 237 g Toluol und 55 g Wasser versetzt, auf 20°C abgekühlt und mit 32%-iger Natronlauge ein pH-Wert von 6 eingestellt. Die untere wäßrige Phase wird abgetrennt und das Lösungsmittel der organischen Phase destillativ entfernt.

Nach HPLC-Methode mit externem Standard wird eine chemische Ausbeute von 90% an N-[(6-Chlorpyridin-3-yl)methyl]-2,2-difluorethan-1-amin bezogen auf eingesetztes 2-Chlor-(5-chlormethyl)pyridin erhalten.

NMR ¹H (CDCl₃) : 5,5 - 5,9 (m, 1H), 2,94 - 3,1 (m, 2 H), 1,26 (br m, NH₂).

### Beispiel 2 (erfindungsgemäß)

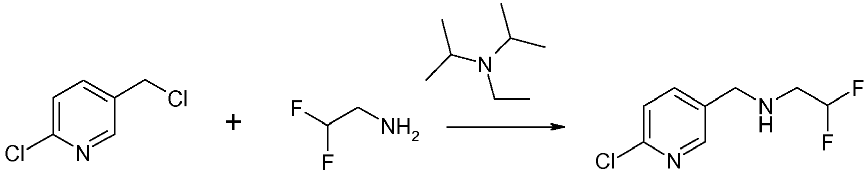

74,4 g (0,90 mol) 2,2-Difluorethylamin (Gehalt: 98%) und 62,6 g (0,48 mol) N, N-Diisopropylethylamin (Gehalt: 99%) werden auf 55°C erhitzt. Zu dieser Mischung werden 49,5 g (0,30 mol) 2-Chlor-(5-chlormethyl)pyridin (Gehalt: 98%) innerhalb von 2,5 Stunden bei dieser Temperatur zugetropft. Die gelbe Lösung läßt man 2 Stunden bei dieser Temperatur nachrühren und destilliert anschließend 72,6 g einer Mischung aus 2,2-Difluorethylamin und N, N-Diisopropylethylamin ab.

Nach GC-Methode mit externem Standard entspricht die Wiedergewinnung an 2,2-Difluorethylamin-Überschuss 0,559 mol (93%) und die Wiedergewinnung N, N-Diisopropylethylamin-Überschuss 0,17 mol (94%).

Der Rückstand wird mit 218 g Toluol und 55 g Wasser versetzt, auf 20°C abgekühlt und mit 32%-iger Natronlauge ein pH-Wert von 6 eingestellt. Die untere wäßrige Phase wird abgetrennt und das Lösungsmittel der organischen Phase destillativ entfernt.

Nach HPLC-Methode mit externem Standard wird eine chemische Ausbeute von 89% an N-[(6-Chlorpyridin-3-yl)methyl]-2,2-difluorethan-1-amin bezogen auf eingesetztes 2-Chlor-(5-chlormethyl)pyridin erhalten.

NMR ¹H (CDCl₃) : 5,5 - 5,9 (m, 1H), 2,94 - 3,1 (m, 2 H), 1,26 (br m, NH₂).

### Beispiel 3 (alternativ)

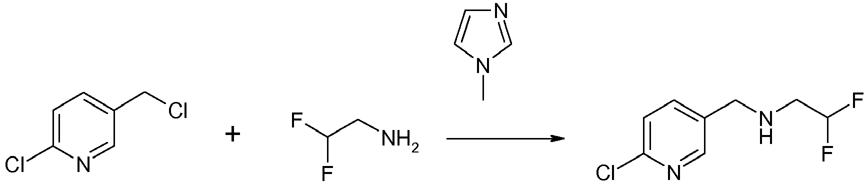

74,4 g (0,90 mol) 2,2-Difluorethylamin (Gehalt: 98%) und 39,8 g (0,48 mol) 1-Methyl-1H-imidazol (Gehalt: 99%) werden auf 55°C erhitzt. Zu dieser Mischung werden 49,5 g (0,30 mol) 2-Chlor-(5-chlormethyl)pyridin (Gehalt: 98%) innerhalb von 2,5 Stunden bei dieser Temperatur zugetropft. Die gelbe Lösung läßt man 2 Stunden bei dieser Temperatur nachrühren und destilliert anschließend 48,4 g 2,2-Difluorethylamin ab.

Nach GC-Methode mit externem Standard entspricht die Wiedergewinnung an 2,2-Difluorethylamin-Überschuss 95,6% des eingesetzten Überschusses.

Der Rückstand wird mit 217 g Butyronitril und 55 g Wasser versetzt, auf 20°C abgekühlt und mit 20%-iger Salzsäure ein pH-Wert von 6 eingestellt. Die untere wäßrige Phase wird abgetrennt und das Lösungsmittel der organischen Phase destillativ entfernt.

Nach HPLC-Methode mit externem Standard wird eine chemische Ausbeute von 71% an N-[(6-Chlorpyridin-3-yl)methyl]-2,2-difluorethan-1-amin bezogen auf eingesetztes 2-Chlor-(5-chlormethyl)pyridin erhalten.

NMR ¹H (CDCl₃) : 5,5 - 5,9 (m, 1H), 2,94 - 3,1 (m, 2 H), 1,26 (br m, NH₂).

### Beispiel 4 (gemäß WO-A-2007/115644)

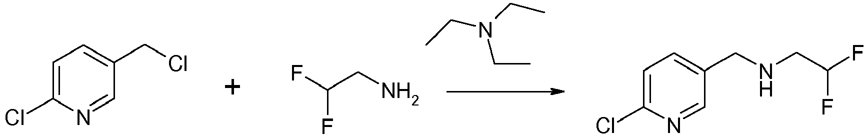

74,4 g (0,90 mol) 2,2-Difluorethylamin (Gehalt: 98%) und 48,5 g (0,48 mol) Triethylamin (Gehalt: 99%) werden auf 55°C erhitzt. Zu dieser Mischung werden 49,5 g (0,30 mol) 2-Chlor-(5-chlormethyl)pyridin (Gehalt: 98%) innerhalb von 2,5 Stunden bei dieser Temperatur zugetropft. Die gelbe Lösung läßt man 2 Stunden bei dieser Temperatur nachrühren und destilliert anschließend 74,0 g einer Mischung aus 2,2-Difluorethylamin und Triethylamin ab.

Nach GC-Methode mit externem Standard entspricht die Wiedergewinnung an 2,2-Difluorethylamin-Überschuss 0,50 mol (83%) und die Wiedergewinnung Triethylamin-Überschuss 0,11 mol (60%)

Der Rückstand wird mit 217 g Toluol und 55 g Wasser versetzt, auf 20°C abgekühlt und mit 20%-iger Salzsäure ein pH-Wert von 6 eingestellt. Die untere wäßrige Phase wird abgetrennt und das Lösungsmittel der organischen Phase destillativ entfernt.

Nach HPLC-Methode mit externem Standard wird eine chemische Ausbeute von 62% an N-[(6-Chlorpyridin-3-yl)methyl]-2,2-difluorethan-1-amin bezogen auf eingesetztes 2-Chlor-(5-chlormethyl)pyridin erhalten.

NMR ¹H (CDCl₃) : 5,5 - 5,9 (m, 1H), 2,94 - 3,1 (m, 2 H), 1,26 (br m, NH₂).

### Ergebnis:

Bei Einsatz von Diisopropylethylamin als tertiärer Stickstoff-Base im erfindungsgemäßen Verfahren werden sehr hohe Ausbeuten von 90 % bzw. 89% erzielt (Beispiele 1 und 2).

Bei Einsatz von Triethylamin als tertiärer Stickstoff-Base im Verfahren gemäß dem Stand der Technik (WO-A-2007/115644) werden dagegen deutlich niedrigere Ausbeuten von nur 62% erzielt (Beispiel 4).

Bei Einsatz von C1-C12-Alkylimidazolen als einer anderen tertiäreren Stickstoff- Base werden Ausbeuten von 71 % erzielt (Beispiel 3 unter Einsatz von 1-Methyl-1H-imidazol).

## Patentansprüche

1. Verfahren zur Herstellung eines 2,2-Difluorethylamins der Formel (III) 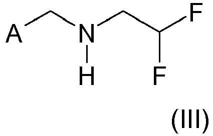 in dem 2,2-Difluorethylamin der Formel (I) 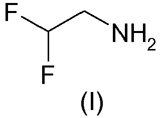 mit einem Halogenid der Formel (II) 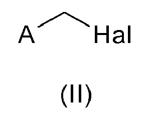 in dem Hal für Chlor, Brom oder Jod steht,
in Gegenwart von Diisopropylethylamin umgesetzt wird, wobei in den Formel (II) und (III)
A für einen Rest Pyrid-2-yl, Pyrid-4-yl oder Pyrid-3-yl steht, der jeweils in 6-Position durch Fluor, Chlor, Brom, Methyl, Trifluormethyl oder Trifluormethoxy substituiert sein kann, oder für Pyridazin-3-yl steht, das in 6-Position durch Chlor oder Methyl substituiert sein kann, oder für einen Rest Pyrazin-3-yl, 2-Chlor-pyrazin-5-yl oder 1,3-Thiazol-5-yl steht, der jeweils in 2-Position durch Chlor oder Methyl substituiert sein kann, oder für einen Rest Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, der durch Fluor, Chlor, Brom, Cyano, Nitro, gegebenenfalls durch Fluor und/oder Chlor substituiertes C₁-C₄-Alkyl, gegebenenfalls durch Fluor und/oder Chlor substituiertes C₁-C₃-Alkylthio, oder gegebenenfalls durch Fluor und/oder Chlor substituiertes C₁-C₃-Alkylsulfonyl, substituiert sein kann, oder für ein Pyrid-3-yl der folgenden Formel 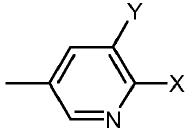
steht, in der
X für Fluor, Chlor, Brom, Iod, C₁-C₆-Alkyl oder C₁-C₆-Halogenalkyl steht und
Y für Fluor, Chlor, Brom, Iod, C₁-C₆-Alkyl, C₁-C₆-Halogenalkyl, C₁-C₆-Halogenalkoxy, Azido oder Cyano steht.

2. Verfahren nach Anspruch 1, in dem das molare Verhältnis von Diisopropylethylamin zu dem eingesetztem Halogenid der Formel (II) im Bereich von 10 bis 0,5 liegt.

3. Verfahren nach Anspruch 1 oder 2, in dem das molare Verhältnis von Halogenid der Formel (II) zum eingesetzten 2,2-Difluorethylamin im Bereich von etwa 1 : 1,5 bis etwa 1 : 20 liegt.

4. Verfahren nach einem der Ansprüche 1 bis 3, in dem nach Beendigung des Verfahrens Diisopropylethylamin und das im Überschuss vorhandene 2,2-Difluorethylamin abgetrennt und dem Verfahren wieder zugeführt werden.

5. Verfahren nach einem der Ansprüche 1 bis 4, mit dem N-[(6-Chlorpyridin-3-yl)methyl]-2,2-difluorethan-1-amin der Formel (III) hergestellt wird, in dem 2-Chlor-(5-chlormethyl)pyridin als Halogenid der Formel (II) verwendet wird.

## Claims

1. Method for preparing a 2,2-difluoroethylamine of the formula (III) 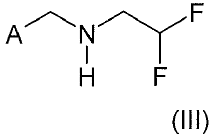 in which 2,2-difluoroethylamine of the formula (I) 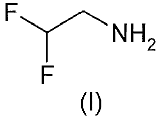 is reacted with a halide of the formula (II) 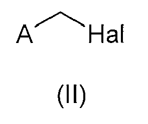 where Hal is chlorine, bromine or iodine,
in the presence of diisopropylethylamine,
where, in the formulae (II) and (III)
A is a pyrid-2-yl, pyrid-4-yl or pyrid-3-yl radical each of which may be substituted in the 6-position by fluorine, chlorine, bromine, methyl, trifluoromethyl or trifluoromethoxy or is pyridazin-3-yl which may be substituted in the 6-position by chlorine or methyl, or is a pyrazin-3-yl, 2-chloropyrazin-5-yl or 1,3-thiazol-5-yl radical which may respectively be substituted in the 2-position by chlorine or methyl, or is a pyrimidinyl, pyrazolyl, thiophenyl, oxazolyl, isoxazolyl, 1,2,4-oxadiazolyl, isothiazolyl, 1,2,4-triazolyl or 1,2,5-thiadiazolyl radical which may be substituted by fluorine, chlorine, bromine, cyano, nitro, C₁-C₄-alkyl optionally substituted by fluorine and/or chlorine, C₁-C₃-alkylthio optionally subsituted by fluorine and/or chlorine, or C₁-C₃-alkylsulphonyl optionally substituted by fluorine and/or chlorine, or is a pyrid-3-yl of the following formula 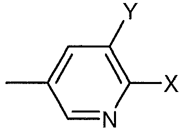
where
X is fluorine, chlorine, bromine, iodine, C₁-C₆-alkyl or C₁-C₆-haloalkyl and
Y is fluorine, chlorine, bromine, iodine, C₁-C₆-alkyl, C₁-C₆-haloalkyl, C₁-C₆-haloalkoxy, azido or cyano.

2. Method according to Claim 1, in which the molar ratio of diisopropylethylamine to the halide of the formula (II) used is in the range from 10 to 0.5.

3. Method according to Claim 1 or 2, in which the molar ratio of halide of the formula (II) to the 2,2-difluoroethylamine used is in the range from approximately 1 : 1.5 to approximately 1 : 20.

4. Method according to any of Claims 1 to 3, in which, after completion of the method, diisopropylethylamine and the 2,2-difluoroethylamine present in excess are removed and are fed again into the method.

5. Method according to any of Claims 1 to 4, with which N-[(6-chloropyridin-3-yl)methyl]-2,2-difluoroethan-1-amine of the formula (III) is prepared, in which 2-chloro-(5-chloromethyl)pyridine is used as halide of the formula (II).

## Revendications

1. Procédé pour la préparation d'une 2,2-difluoroéthylamine de formule (III) 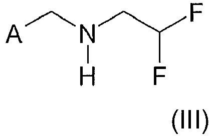 dans lequel on transforme de la 2,2-difluoroéthylamine de formule (I) 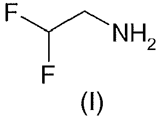 avec un halogénure de formule (II) 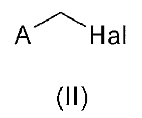 dans laquelle Hal représente chlore, brome ou iode, en présence de diisopropyléthylamine, où, dans les formules (II) et (III)
A représente un radical pyrid-2-yle, pyrid-4-yle ou pyrid-3-yle, qui peut à chaque fois être substitué en 6ème position par fluor, chlore, brome, méthyle, trifluorométhyle ou trifluorométhoxy ; ou représente pyridazin-3-yle qui peut être substitué, en 6ème position, par chlore ou méthyle ; ou représente un radical pyrazin-3-yle, 2-chloropyrazin-5-yle ou 1,3-thiazol-5-yle, qui peut à chaque fois être substitué en 2ème position par chlore ou méthyle ; ou représente un radical pyrimidinyle, pyrazolyle, thiophényle, oxazolyle, isoxazolyle, 1,2,4-oxadiazolyle, isothiazolyle, 1,2,4-triazolyle ou 1,2,5-thiadiazolyle, qui peut être substitué par fluor, chlore, brome, cyano, nitro, par C₁-C₄-alkyle le cas échéant substitué par fluor et/ou chlore, par C₁-C₃-alkylthio le cas échéant substitué par fluor et/ou chlore ou par C₁-C₃-alkylsulfonyle le cas échéant substitué par fluor et/ou chlore ; ou représente pyrid-3-yle de la formule suivante 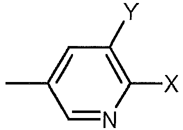
dans laquelle
X représente fluor, chlore, brome, iode, C₁-C₆-alkyle ou C₁-C₆-halogénoalkyle et
Y représente fluor, chlore, brome, C₁-C₆-alkyle, C₁-C₆-halogénoalkyle, C₁-C₆-halogénoalcoxy, azoture ou cyano.

2. Procédé selon la revendication 1, dans lequel le rapport molaire de diisopropyléthylamine à l'halogénure utilisé de formule (II) se situe dans la plage de 10 à 0,5.

3. Procédé selon la revendication 1 ou 2, dans lequel le rapport molaire de l'halogénure de formule (II) à la 2,2-difluoroéthylamine utilisée se situe dans la plage d'environ 1:1,5 à environ 1:20.

4. Procédé selon l'une quelconque des revendications 1 à 3, dans lequel, après la fin du procédé, la diisopropyléthylamine et la 2,2-difluoroéthylamine présente en excès sont séparées et de nouveau introduites dans le procédé.

5. Procédé selon l'une quelconque des revendications 1 à 4, par lequel on prépare de la N-[(6-chloropyridin-3-yl)méthyl]-2,2-difluoroéthan-1-amine de formule (III), dans lequel on utilise de la 2-chloro-(5-chlorométhyl)pyridine comme halogénure de formule (II).
